(19) Europäisches Patentamt
European Patent Office
Office européen des brevets

(11) Numéro de publication: **0 243 281 B1**

(12) # FASCICULE DE BREVET EUROPEEN

(45) Date de publication de fascicule du brevet: **29.01.92**

(51) Int. Cl.5: **C07C 67/465**, C07C 69/593, C07C 69/587, B01J 31/28

(21) Numéro de dépôt: **87420078.5**

(22) Date de dépôt: **20.03.87**

(54) **Procédé de dimérisation d'un acrylate d'alkyle inférieur et composition catalytique.**

(30) Priorité: **27.03.86 FR 8604644**

(43) Date de publication de la demande: **28.10.87 Bulletin 87/44**

(45) Mention de la délivrance du brevet: **29.01.92 Bulletin 92/05**

(84) Etats contractants désignés: **AT BE CH DE ES FR GB IT LI LU NL SE**

(56) Documents cités:
**FR-A- 2 079 319**
**FR-A- 2 524 341**
**US-A- 4 318 860**
**US-A- 4 451 665**

**TETRAHEDRON LETTERS, no. 4, 1979, pages 343-344, Pergamon Press Ltd., GB; G. OEHME et al.: "Zur Kenntnis der katalytischen Dimerisation von Acrylsaurederivaten mit Hilfe von palladium-Komplexen II. Die Aktivierung von Dichloro-nitril- und phosphin-palladium(II)-Komplexen mit Silbertetrafluoroborat zur Dimerisation von Methylagrylat"**

(73) Titulaire: **RHONE-POULENC CHIMIE**
**25, quai Paul Doumer**
**F-92408 Courbevoie Cédex(FR)**

(72) Inventeur: **Grenouillet, Pierre**
**Résidence Hautefeuille 19 A, rue Gambetta**
**F-69 279 Fontaines sur Saone(FR)**
Inventeur: **Neibecker, Denis**
**77, rue R. Salengro**
**F-54230 Neuves-Maisons(FR)**
Inventeur: **Tkatchenko, Igor**
**25 D, rue Lassagne**
**F-69300 Caluire(FR)**

(74) Mandataire: **Varnière-Grange, Monique et al**
**RHONE-POULENC INTERSERVICES Service Brevets Chimie Centre de Recherches de Saint-Fons B.P. 62**
**F-69192 Saint-Fons Cédex(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention se rapporte à un procédé de dimérisation catalytique d'un acrylate d'alkyle en présence de certains complexes du palladium et plus particulièrement à la dimérisation catalytique d'esters alkyliques de l'acide acrylique pour produire des diesters alkyliques de l'acide dihydromuconique.

Certains dérivés du palladium sont connus pour catalyser la dimérisation des acrylates d'alkyles. Ainsi, le chlorure de bis(benzonitrile) palladium II, $(C_6H_5CN)_2$ $PdCl_2$, a été mis en oeuvre pour dimériser l'acrylate de méthyle par Barlow et coll., J. Organometal. Chem., (1970) 21, 215. Le système est néanmoins peu efficace puisque, dans ce travail antérieur, une conversion de 67 % pour une sélectivité de 93 % en dimères dont 90 % en dimère linéaire ont été obtenus en 23 heures à 113 ° C. La vitesse de réaction a pu être améliorée par ajoût au milieu réactionnel de tétrafluoroborate d'argent, ($AgBF_4$) comme cela est indiqué par OEHME et coll. dans Tetrahedron Letters, (1979) 4, 343. Cette vitesse de réaction a également pu être améliorée par ajoût de p-benzoquinone, d'après PRACEJUS et coll. Dans Z. Chem., (1980) 20, 24.

On connaît aussi l'efficacité des complexes cationiques du palladium de formule $Pd(NCMe)_4(BF_4)_2$ en particulier en présence de $LiBF_4$ anhydre puisqu'à 40 ° C en 30 heures on obtient après distillation 93 % de rendement en dimères de l'acrylate de méthyle, la sélectivité en isomère $\Delta^2$ trans étant de L'ordre de 93-96 % [cf. NUGENT et Coll. dans J. Org. Chem., (1983), 48, 5364-5366].

On connaît par ailleurs (cf. FR 2 079 319) la co-dimérisation du butadiène -1,3 et de l'acrylate de méthyle pour former des heptadiénoates de méthyle catalysée par un mélange d'un chlorure de palladium du type $\pi$-allyle, d'une phosphine et d'un sel d'argent.

Il a également été proposé (cf. FR 2 524 341) de catalyser la réaction de dimérisation d'un acrylate d'alkyle ou de codimérisation de ce type de dérivés avec un composé diènique conjugué par un complexe allylique cationique du palladium modifié par l'adjonction d'une phosphine, les complexes en cause étant préparés à partir de composé du palladium et de la dibenzylidèneacétone et d'un sel d'allyloxytris (diméthylamino)phosphonium et de cyclooctadiène-1,5.

Pour résumer, toutes ces réactions, lorsqu'elles mettent en oeuvre des complexes aisément accessibles du palladium tels :

$pdCl_2(PhCN)_2$ ou $[pd(C_3H_5)Cl]_2$

sont généralement lentes et/ou peu sélectives. L'amélioration de l'activité et/ou de la sélectivité de ces réactions s'accompagne d'un accroissement de la complexité des composés du palladium et de ce fait du coût de leur préparation et de difficultés accrues dans leur mise en oeuvre, causées notamment par leur instabilité.

Il s'avérait donc nécessaire de proposer des systèmes catalytiques à la fois efficaces et plus économiques que ceux antérieurement préconisés.

La présente invention a donc pour objet un procédé de dimérisation catalytique d'un acrylate d'alkyle inférieur par mise en contact à une température comprise entre 50 et 250 ° C d'un acrylate d'alkyle inférieur en présence d'un système catalytique comprenant du palladium ou un composé du palladium, caractérisé en ce que le système catalytique est formé à partir d'au moins :

a) une source de palladium non halogénée

b) un composé du phosphore (III) de formule générale (I) :

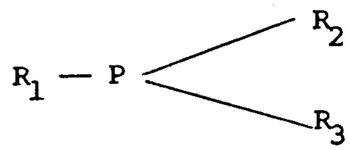

(I)

dans laquelle :

$R_1$, $R_2$ et $R_3$ représentent indépendamment un radical alkyle, cycloalkyle, aryle, alcoxy, cycloalcoxy ou aryloxy,

l'un des radicaux $R_1$, $R_2$ ou $R_3$ pouvant en outre représenter un radical monovalent de formule générale (II) :

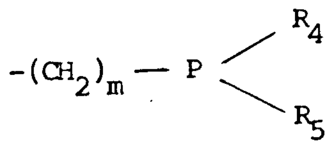 (II)

dans laquelle:

- m est un entier compris entre 1 et 4 inclus,
- $R_4$ et $R_5$ représentent indépendamment un radical alkyle, cycloalkyle, aryle, alcoxy, cycloalcoxy ou aryloxy,

et

c) d'au moins un hydracide HY dont l'anion associé $Y^-$ présente un caractère non coordinant vis à vis des ions palladium.

L'invention a également pour objet la composition catalytique utilisable lors de la mise en oeuvre dudit procédé.

La matière de départ est un acrylate d'alkyle inférieur, c'est à dire dont le reste alkyle comporte de 1 à 8 atomes de carbone ; la dimension du groupe alkyle n'est pas critique et on recourt plus particulièrement à l'acrylate de méthyle ou à l'acrylate d'éthyle en raison de leur plus grande disponibilité. Le groupement alkyle peut comporter des substituants n'interférant pas avec la réaction recherchée.

Bien entendu on peut utiliser comme matière de départ des produits du commerce non nécessairement purs.

Le système catalytique selon l'invention est formé à partir d'au moins une source de palladium non halogénée. Cette source de palladium, précurseur de l'entité catalytiquement active, peut être choisie parmi les diverses formes de palladium (0) ou de palladium (II) non halogénées. Parmi les formes de palladium (II) susceptibles de convenir à la mise en oeuvre du procédé selon l'invention, on peut citer les sels d'acides organiques tels l'acétate de palladium (II), le formiate de palladium (II), le propionate de palladium (II), l'octanoate de palladium (II), l'éthylhexanoate de palladium (II) ; les sels d'acides minéraux tel le nitrate de palladium (II), des complexes $\pi$- allyliques de palladium (II) tel le diacétate de ($\pi$-allyl)palladium ; l'acétylacétonate de palladium (II).

On préconisera l'emploi de l'acétate de palladium (II) ou de l'acétylacétonate de palladium (II), notamment en raison de leur plus grande disponibilité.

Parmi les sources de palladium (0) susceptibles de convenir à la mise en oeuvre de la présente invention on peut citer le noir de palladium, le palladium déposé sur un support tel le charbon actif ou le gel de silice, les complexes du palladium et d'une trialkyl- ou triarylphosphine tel le tétrakis-(triphénylphosphine)palladium.

Les composés du palladium (0) et de la dibenzylidèneacétone qui répondent à la formule générale (III) ci-après conviennent particulièrement bien à la mise en oeuvre de la présente invention :

$Pd_x(dba)_y$ (III)

dans laquelle :
x est égal à 1 ou 2
dba représente un coordinat dibenzylidèneacétone,
y est égal à 2 ou à 3, y étant nécessairement égal à 3 lorsque x vaut 2.

Ces composés sont d'un accès facile. Il peuvent être obtenus aisément par la réduction du chlorure de palladium en présence de dibenzylidèneacétone (dba) selon l'un quelconque des modes opératoires décrits par Y. ISHII et coll. dans Chem. Comm. , 1970, p 1065.

Les complexes $Pd(dba)_2$, $Pd_2(dba)_3$ et $Pd(dba)_3$ et leurs mélanges peuvent être utilisés indifféremment.

La concentration du palladium dans le milieu réactionnel peut varier dans de larges limites. Une quantité d'au moins 0,1 millimole par mole d'acrylate paraît nécessaire pour obtenir un taux de transformation suffisant. On n'observe pas d'avantage à dépasser la quantité de 3 millimoles par mole d'acrylate. Une quantité comprise entre 0,2 et 1,5 millimole par mole d'acrylate paraît être un compromis acceptable entre le coût et l'efficacité du système catalytique.

Le système catalytique utilisé dans le cadre du présente procédé est formé également à partir d'au moins un composé du phosphore (III) de formule générale (I) :

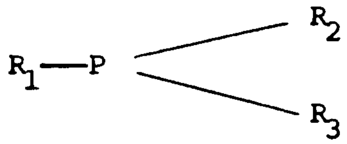 (I)

dans laquelle :
$R_1$, $R_2$ et $R_3$ ont la signification donnée précédemment, l'un des radicaux $R_1$ à $R_3$ pouvant en outre représenter un radical monovalent de formule générale (II) :

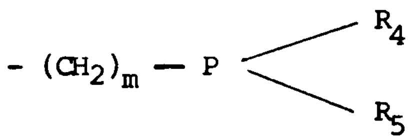 (II)

m, $R_4$ et $R_5$ ayant été également précédemment définis.

Plus spécifiquement $R_1$, $R_2$ et $R_3$ identiques ou différents peuvent représenter :

- un radical alkyle contenant au maximum 8 atomes de carbone tels que les radicaux méthyle, éthyle, propyle, isopropyle, butyle, t-butyle et n-octyle,
- un radical cycloalkyle contenant de 5 à 7 atomes de carbone tel qu'un radical cyclohexyle ou cycloheptyle,
- un radical aryle contenant de 6 à 12 atomes de carbone tels les radicaux phényle, p-toluyle, biphénylyle, naphtyle,
- un radical alcoxy comportant au plus 8 atomes de carbone tels les radicaux méthoxy et éthoxy,
- un radical aryloxy comportant de 6 à 12 atomes de carbone tel le radical phénoxy.

A titre d'exemple de phosphines ou phosphites répondant à la formule (I) ci-avant on peut citer la tributylphosphine, la tricyclohexylphosphine, le triéthylphosphite, la diméthylphénylphosphine et le triphénylphosphite.

A titre d'exemple de diphosphines répondant à la formule (II) on peut citer :
le bis(diméthylphosphino)méthane,
le bis(diphénylphosphino)méthane,
le bis(diphénylphosphino)éthane,
le bis(diphénylphosphino)propane,
le bis(diphénylphosphino)butane, et
le bis(dicyclohexylphosphino)éthane.

On préfère les phosphines comportant au moins un radical alkyle ou cycloalkyle et plus particulièrement les trialkylphosphines.

On recourt avantageusement à la tributylphosphine. La quantité de composé de phosphore (III) est en général telle que le rapport molaire P/Pd soit compris entre 1 et 15 et, de préférence entre 1 et 3.

Le système catalytique utilisé dans le cadre de la présente invention est formé à partir des deux types de composés (Pd, P) précités auxquels il faut encore adjoindre un hydracide HY dont l'anion associé $Y^-$ présente un caractère non coordinant vis à vis des ions palladium. Les hydracides sont généralement des acides forts dont l'anion $Y^-$ est choisi dans le groupe constitué par les anions :

$$C_nF_{2n+1}CO_2^-,\ C_nF_{2n+1}SO_3^-,\ 1/2\ SO_4^{=},\ ClO_4^-,\ PF_6^-,\ SbF_6^-,\ BF_4^-,\ 1/2\ SiF_6^{=},$$

n étant un entier compris entre 1 et 4 inclus.

L'acide tétrafluoroborique convient plus particulièrement à la préparation dudit système catalytique.

Le rapport $H^+/Pd$ est en général compris entre 1 et 30, et de préférence entre 1 et 10. Ce rapport est lié au rapport molaire P/Pd ; de bons résultats étant obtenus pour un rapport $H^+/P$ de l'ordre de 2 à 5 environ.

La préparation du système catalytique s'effectue par addition successive, le cas échéant en milieu solvant, du dérivé de phosphore (III) et de l'hydracide à la source de palladium. Une variante de cette préparation peut résider dans la synthèse préalable d'un sel d'hydrogénophosphonium de formule :

$[HPR_1R_2R_3]^+Y^-$

par addition, en milieu solvant, le cas échéant, de l'hydracide sur le composé du phosphore (III) lorsque ce dernier est suffisamment basique. Ceci est notamment possible avec la tributylphosphine, la tricyclohexylphosphine et la diméthylphenylphosphine. Bien entendu les différentes proportions Pd,P,$H^+$ indiquées précédemment restent applicables.

Le solvant de la réaction peut être le substrat mis en oeuvre dans la réaction catalytique. Des solvants n'interférant pas avec la réaction catalytique elle-même conviennent également. A titre d'exemple de tels solvants on peut citer les hydrocarbures saturés ou aromatiques, les éthers, les esters, les halogénures aliphatiques et aromatiques.

La préparation du système catalytique est conduite à une température comprise entre la température ambiante (environ 20° C) et environ 100° C ; la plage de températures comprise entre 40 et 100° C s'avère plus avantageuse.

La réaction de dimérisation est généralement effectuée à une température comprise entre 50 et 250° C de préférence au voisinage de 70 à 200° C, pendant une durée comprise entre 10 minutes et 72 heures, et de préférence, entre 30 minutes et 20 heures.

Les produits de réaction sont généralement récupérés par distillation après neutralisation du milieu réactionnel lorsqu'un excès d'hydracide est employé et, le cas échéant, une première distillation pour assurer l'élimination d'un tiers solvant lorsque cela est nécessaire.

Le procédé convient particulièrement bien à la dimérisation des acrylates d'alkyles, notamment de l'acrylate de méthyle ou d'éthyle. Les diesters ainsi obtenus sont des intermédiaires utiles pour la fabrication de l'acide adipique.

Les exemples ci-après illustrent l'invention.

Dans les exemples les conventions suivantes sont utilisées :

DELTA-2 désigne le $\Delta^2$-dihydromuconate de méthyle
DELTA-3 désigne le $\Delta^3$-dihydromuconate de méthyle
ME-2G désigne le méthylène-2 glutarate de méthyle
polym. désigne des polymères.
tr. désigne des traces.

EXEMPLES 1 A 17

Ces exemples illustrent la dimérisation de l'acrylate de méthyle selon le mode opératoire général suivant :

Dans un tube de Schlenk muni d'un barreau aimanté et purgé à l'argon, on introduit successivement :

- l'équivalent de 0,2 millimole (mmol) en palladium d'un mélange 1:3 des complexes $Pd(dba)_2$ et $Pd_2(dba)_3$,
- de l'acrylate de méthyle commercial, dégazé,
- de la tributylphosphine ($PBu_3$) redistillée sous argon,
- de l'acide tétrafluoroborique en solution dans le diéthyléther, dont le titre a été déterminé au préalable.

Le tube est alors fermé puis porté sous agitation à la température de 80° C pendant le temps souhaité. Il est ensuite ramené à la température ambiante et le milieu réactionnel est alors neutralisé par ajoût de 0,5 g de bicarbonate de sodium. On distille sous pression réduite (3 torr) et on récupère la fraction liquide.

Le distillat ainsi obtenu est alors analysé par chromatographie en phase gazeuse avec comme étalon interne le malonate de méthyle, avec une colonne : 2 m x 1/8" Carbowax 20 M (10 %) sur Chromosorb PAW 60-80, la température de l'injecteur est de 250° C, celle du four de 150° C, celle du détecteur à ionisation de flamme de 250° C et on utilise l'azote (25ml/mn) comme gaz vecteur.

Les conditions particulières ainsi que les résultats obtenus figurent dans le tableau I ci-après.

TABLEAU I

| Dimérisation de l'acrylate de méthyle - Influence des proportions $Pd/P/H^+$ et du temps (température de réaction : 80 °C) | | | | | | | | | | |
|---|---|---|---|---|---|---|---|---|---|---|
| N° Ex. | [Pd(0)] mmol | $[PBu_3]$ mmol | $[HBF_4]$ mmol | Acrylate de méthyle mmol | Temps h | Conversion % | SELECTIVITES % | | | |
| | | | | | | | DELTA-2 | DELTA-3 | ME-2G | autres |
| 1 | 0,2 | 0,2 | 0,2 | 157 | 20 | 4 | 94 | 1 | 1 | 4 |
| 2 | 0,2 | 0,2 | 0,4 | 152 | 20 | 39 | 95 | tr. | 1,5 | 3 |
| 3 | 0,2 | 0,2 | 0,6 | 157 | 20 | 53 | 95 | tr. | 1 | 4 |
| 4 | 0,2 | 0,2 | 0,8 | 147 | 20 | 40 | 94 | tr. | 1,5 | 4 |
| 5 | 0,2 | 0,2 | 1 | 153 | 20 | 21 | 95 | tr. | 2 | 3 |
| 6 | 0,2 | 0,4 | 0,4 | 156 | 20 | 12 | 94 | tr. | 1,5 | 4,5 |
| 7 | 0,2 | 0,4 | 0,6 | 152 | 20 | 55 | 95 | tr | 1 | 4 |
| 8 | 0,2 | 0,4 | 0,8 | 154 | 20 | 87 | 96 | tr. | 1 | 3 |
| 9 | 0,2 | 0,4 | 1 | 149 | 20 | 87 | 96 | tr. | 1 | 4 |
| 10 | 0,2 | 0,6 | 0,8 | 136 | 20 | 55 | 95 | tr. | 2 | 3 |
| 11 | 0,2 | 0,6 | 1 | 145 | 20 | 53 | 95 | 1 | 1 | 3 |
| 12 | 0,2 | 0,4 | 0,8 | 153 | 5 | 64 | 96 | tr. | 1 | 3 |
| 13 | 0 | 0,2 | 0,2 | 150 | 20 | 95 | | | | polym. |
| 14 | 0,2 | 0 | 0,2 | 136 | 20 | 0 | | | | - |
| 15 | 0,2 | 0 | 0,4 | 141 | 20 | 0 | | | | - |
| 16 | 0,2 | 0,2 | 0 | 140 | 20 | 0 | | | | - |
| 17 | 0 | 0 | 0,2 | 150 | 20 | 0 | | | | - |

Dans l'exemple 13 (qui ne fait pas partie de l'invention) il est montré qu'en l'absence de palladium on ne forme que les polymères.

Dans l'exemple 14 (qui ne fait pas partie de l'invention) il est montré qu'en l'absence de composé du phosphore (III) on n'observe aucune transformation de l'acrylate.

Dans l'exemple 15 (qui ne fait pas partie de l'invention) il est montré qu'en l'absence de composé du phosphore (III) et en présence d'une quantité accrue d'hydracide on n'observe aucune transformation de l'acrylate.

Dans l'exemple 16 (qui ne fait pas partie de l'invention) il est montré qu'en l'absence d'hydracide on n'observe aucune transformation de l'acrylate.

Dans l'exemple 17 (qui ne fait pas partie de l'invention) il est montré que la seule présence de l'hydracide ne suffit pas pour assurer une quelconque transformation de l'acrylate.

EXEMPLES 18 ET 19

Ces exemples mettent en oeuvre le préformage du sel $[HPBu_3]BF_4$.

Dans un tube de Schlenk muni d'un barreau aimanté et purgé à l'argon, on place successivement :

- l'équivalent de 0,2 millimole en palladium du mélange de complexes précité ;
- l'acrylate de méthyle commercial, dégazé,
- du tétrafluoroborate d'hydrogénotributylphosphonium formé par la réaction de quantités équivalentes de tributylphosphine et d'acide tétrafluoroborique en solution dans le diéthyléther et, le cas échéant, de l'acide tétrafluoroborique en solution dans le diéthyléther. Le tube de Schlenk est fermé puis agité à 80 °C pendant 20 heures. Le traitement des mélanges réactionnels selon la procédure décrite précédemment conduit aux résultats consignés dans le tableau II ci-après.

TABLEAU II

| Dimérisation de l'acrylate de méthyle. Préformage du sel $[HPBu_3]BF_4$ (réaction à 80 °C, en 20 h) | | | | | | | | | |
|---|---|---|---|---|---|---|---|---|---|
| N° Ex. | [Pd(0)] mmol | $[HPBu_3]BF_4$ mmol | $[HBF_4]$ mmol | Acrylate de méthyle mmol | Conversion % | SELECTIVITES % | | | |
| | | | | | | DELTA-2 | DELTA-3 | ME-2G | autres |
| 18 | 0,2 | 0,2 | 0,2 | 156 | 23 | 96 | tr. | 1 | 3 |
| 19 | 0,2 | 0,4 | 0 | 163 | 12 | 95 | tr. | 1,5 | 3,5 |

EXEMPLE 20

Cet exemple illustre l'effet de la concentration du précurseur catalytique.

Dans un tube de Schlenk muni d'un barreau aimanté et purgé à l'argon, on place successivement :

- l'équivalent de 0,1 milliatome-gramme en palladium du mélange de complexes précité,
- l'acrylate de méthyle commercial dégazé (13,8 ml : soit 153 mmol),
- la tributylphosphine ($PBu_3$) (40 mg ; soit 0,2 mmol),
- l'acide tétrafluoroborique en solution 9,3 N dans le diéthyléther (0,05 ml ; soit 0,4 mmol), Le tube de Schlenk est fermé puis agité à 80 °C pendant 20 heures. Le traitement du mélange réactionnel selon la procédure décrite précédemment conduit à 41 % de conversion de l'acrylate de méthyle. Les sélectivités observées sont les suivantes :
- $\Delta^2$-dihydromuconate de méthyle : supérieure à 95 %,
- Méthylène-2 glutarate de méthyle : 1 %,
- $\Delta^3$-dihydromuconate de méthyle : traces
- dimères non identifiés : 3 %.

EXEMPLE 21 :

Cet exemple illustre l'emploi de l'acétate de palladium dans la dimérisation de l'acrylate de méthyle.

Dans un tube Schlenk muni d'un barreau aimanté et purgé à l'argon on place successivement

- de l'acétate de palladium (origine : Johnson-Matthey ; 22,5 mg ; 0,1 mmol),
- de l'acrylate de méthyle commercial dégazé (13,8 ml ; 153 mmol),
- de la tributylphosphine (60 mg ; 0,3 mmol),
- de l'acide tétrafluoroborique en solution 9,3 N dans le diéthyléther (0,075 ml ; 0,6 mmol).

Le tube Schlenk est fermé, puis agité à 80 °C pendant 5 heures. Le traitement du mélange réactionnel selon la procédure décrite précédemment conduit à 52 % de conversion de l'acrylate de méthyle (fréquence de rotation : 76 $h^{-1}$). La sélectivité en $\Delta^2$-dihydromuconate de méthyle est supérieure à 95 %.

EXEMPLE 22 :

Cet exemple illustre l'emploi de l'acétylacétonate de palladium dans la dimérisation de l'acrylate de méthyle.

Dans un tube Schlenk muni d'un barreau aimanté et purgé à l'argon on place successivement

- de l'acétylacétonate de palladium (préparé selon la demande de brevet allemand n° 2 904 235 ; 30,5 mg ; 0,1 mmol),
- de l'acrylate de méthyle commercial dégazé (14 ml ; 155 mmol),
- de la tributylphosphine (40 mg ; 0,2 mmol),
- de l'acide tétrafluoroborique en solution 9,3 N dans le diéthyléther (0,125 ml ; 1,0 mmol).

Le tube Schlenk est fermé, puis agité à 80 °C pendant 5 heures. Le traitement du mélange réactionnel selon la procédure décrite précédemment conduit à 67 % de conversion de acrylate de méthyle (fréquence de rotation : 88 $h^{-1}$). La sélectivité en

$\Delta^2$-dihydromuconate de méthyle est supérieure à 95 %.

EXEMPLE 23 :

Cet exemple illustre la dimérisation de l'acrylate de méthyle selon un autre mode opératoire :

Dans un autoclave en acier inoxydable (Z 8 CNDT 1712) préalablement purgé à l'argon, on charge :

- acrylate de méthyle 13,2 g (154 mmol)
- $Pd(dba)_2$ 0,2 mmol
- $PBu_3$ 0,4 mmol
- $HBF_4$ 0,8 mmol

L'autoclave est fermé hermétiquement puis placé dans un four agité par secousses ; la température est régulée à 80° C, la durée de la réaction est de 20 heures. L'autoclave est alors refroidi, on obtient une masse réactionnelle orange et limpide. On n'observe pas de palladium métallique dans la masse réactionnelle. L'analyse montre qu'il s'est formé des hexènedioates de méthyle (rendement de 49 %) et du méthylène-2 pentanedioate de méthyle (6,4 %).

EXEMPLE 24 :

On opère de la même façon que dans l'exemple 25 à ceci près que la température de réaction est de 120° C et la durée de 3 heures. On obtient une masse réactionnelle orange limpide ; on n'observe pas de formation de palladium métallique. Le rendement en hexènedioates de méthyle est de 58 %.

EXEMPLE 25 :

On opère comme dans l'exemple 25 à ceci près que le palladium est introduit sous forme de $Pd(acac)_2$ (acac : acétylacétonate) que la température est de 100° C et la durée de réaction de 3 heures. On obtient une masse réactionnelle jaune limpide. On n'observe pas de formation de palladium métallique. Le rendement en hexènedioates de méthyle est de 49 %.

EXEMPLES 26 A 30 :

Cette série d'essais illustre l'influence de la température ; elle est réalisée selon un mode opératoire décrit pour l'exemple 25

Dans un autoclave en acier inoxydable (Z 8 CNDT 1712) préalablement purgé à l'argon, on charge :

- $Pd(acac)_2$ 0,4 mmol
- acrylate de méthyle 161 mmol
- et une solution, dans l'acrylate de méthyle de $PBu_3$ 0,8 mmol
- et $HBF_4 . Et_2O$ 1,6 mmol

L'autoclave est fermé hermétiquement puis placé dans un four agité par secousses, préchauffé à 215° C ; la température est régulée à 190° C, la durée de la réaction est de 15 minutes. L'autoclave est alors refroidi. On obtient une masse réactionnelle orange limpide, avec quelques traces de palladium métallique en suspension (pour des températures de travail plus basses, on n'observe pas ce phénomène). L'analyse montre qu'il s'est formé des hexènedioates de méthyle (rendement de 84 %) et du méthylène-2 glutarate de méthyle (rendement de 2,6 %). Les conditions particulières ainsi que les résultats obtenus pour diverses températures sont rassemblés dans le tableau III ci-après :

Tableau III

| Ex n° | T°C | Durée en h | Rendement (%) hexènedioates | (%)Rendement ME-2G |
|---|---|---|---|---|
| 26 | 100 | 3 | 37 | 0,6 |
| 27 | 120 | 3 | 67 | 1,4 |
| 28 | 140 | 1 | 95,5 | 2 |
| 29 | 160 | 0,5 | 90 | 2 |
| 30 | 190 | 0,25 | 84 | 2,6 |

EXEMPLE 31 :

On reproduit l'exemple 28 ci-avant en divisant la charge de palladium par deux. Toutes conditions égales par ailleurs les rendements sont respectivement les suivants :

- hexènedioates de méthyle : 16 %

- méthylène-2 glutarate de méthyle : 0,3 %

## Revendications

1. Procédé de dimérisation catalytique d'un acrylate d'alkyle inférieur par mise en contact à une température comprise entre 50 et 250° C d'un acrylate d'alkyle inférieur en présence d'un système catalytique comprenant du palladium ou un composé du palladium, caractérisé en ce que le système catalytique est formé à partir d'au moins :
   a) une source de palladium non halogénée,
   b) un composé du phosphore (III) de formule générale (I) :

   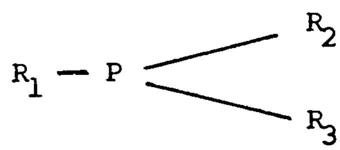
   (I)

   dans laquelle :
   $R_1$, $R_2$ et $R_3$ représentent indépendamment un radical alkyle, cycloalkyle, aryle, alcoxy, cycloalcoxy ou aryloxy,
   l'un des radicaux $R_1$, $R_2$ ou $R_3$ pouvant en outre représenter un radical monovalent de formule générale (II) :

   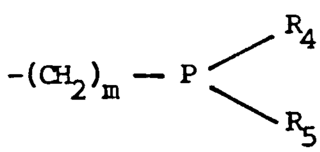
   (II)

   dans laquelle:
   - m est un entier compris entre 1 et 4 inclus,
   - $R_4$ et $R_5$ représentent indépendamment un radical alkyle, cycloalkyle, aryle, alcoxy, cycloalcoxy ou aryloxy,

   et
   c) d'au moins un hydracide HY dont l'anion associé $Y^-$ présente un caractère non coordinant vis à vis des ions palladium.

2. Procédé de dimérisation catalytique d'un acrylate d'alkyle inférieur par mise en contact à une température comprise entre 50 et 250° C d'un acrylate d'alkyle inférieur en présence d'un système catalytique comprenant du palladium ou un composé du palladium, caractérisé en ce que le système catalytique est formé à partir d'au moins :
   a) une source de palladium non halogénée , et
   b) d'au moins un sel d'hydrogénophosphonium de formule :

   $[HPR_1R_2R_3]^+Y^-$

   et le cas échéant d'un hydracide HY, $R_1$, $R_2$, $R_3$ et $Y^-$ ayant la signification donnée dans la revendication 1.

3. Procédé selon la revendication 1 ou 2, caractérisé en ce que le système catalytique est formé en milieu solvant.

4. Procédé selon l'une quelconque des revendications précédentes, caractérisé en ce que $Y^-$, anion associé à l'hydracide est choisi dans le groupe constitué par les anions :

$C_nF_{2n+1}CO_2^-$, $C_nF_{2n+1}SO_3^-$, 1/2 $SO_4^=$, $ClO_4^-$, $PF_6^-$, $SbF_6^-$, $BF_4^-$, 1/2 $SiF_6^=$,

n étant un entier compris entre 1 et 4 inclus.

**5.** Procédé selon l'une quelconque des revendications précédentes, caractérisé en ce que $Y^-$, anion associé à l'hydracide est un anion tetrafluoroborate.

**6.** Procédé selon l'une quelconque des revendications précédentes, caractérisé en ce que le rapport molaire du phosphore au palladium est compris entre 1 et 3.

**7.** Procédé selon l'une quelconque des revendications précédentes, caractérisé en ce que le rapport molaire des ions $H^+$ au palladium est compris entre 1 et 10.

**8.** Procédé selon l'une quelconque des revendications 1, 3 à 7 ci-avant, caractérisé en ce que la phosphine utilisée est la tributylphosphine.

**9.** Procédé selon l'une quelconque des revendications 2 à 8 ci-avant, caractérisé en ce que le sel d'hydrogénophosphonium utilisé est le tétrafluoroborate d'hydrogénotributylosphonium.

**10.** Procédé selon l'une quelconque des revendications précédentes, caractérisé en ce que la source de palladium est choisie parmi les composés du palladium (0) de formule générale (III) :

$Pd_x(dba)_y$ (III)

dans laquelle :
x est égal à 1 ou 2
dba représente un coordinat dibenzylidèneacétone,
y est égal à 2 ou à 3, y étant nécessairement égal à 3 lorsque x vaut 2.

**11.** Procédé selon l'une quelconque des revendications 1 à 9 ci-avant, caractérisé en ce que la source de palladium est l'acétate de palladium.

**12.** Procédé selon l'une quelconque des revendications 1 à 9 ci-avant, caractérisé en ce que la source de palladium est l'acétylacétonate de palladium.

**Claims**

**1.** Process for the catalytic dimerization of a lower alkyl acrylate by bringing a lower alkyl acrylate into contact with a catalytic system comprising palladium or a palladium compound, at a temperature of between 50 and 250 °C, wherein the catalytic system is formed from at least:
a) one nonhalogenated palladium source,
b) one phosphorus(III) compound of general formula (I):

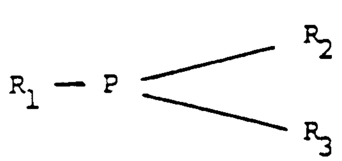

(I)

in which:

$R_1$, $R_2$ and $R_3$ independently represent an alkyl, cycloalkyl, aryl, alkoxy, cycloalkoxy or aryloxy radical, it being possible for one of the radicals $R_1$, $R_2$ or $R_3$ to additionally represent a monovalent

radical of general formula (II):

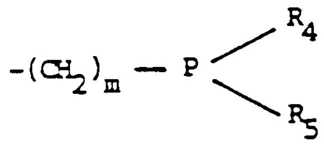 (II)

in which:

m is an integer of between 1 and 4 inclusive,

$R_4$ and $R_5$ independently represent an alkyl, cycloalkyl, aryl, alkoxy, cycloalkoxy or aryloxy radical, and

c) at least one hydracid HY, the associated anion $Y^-$ of which does not coordinate with palladium ions.

**2.** Process for the catalytic dimerization of a lower alkyl acrylate by bringing a lower alkyl acrylate into contact with a catalytic system comprising palladium or a palladium compound, at a temperature of between 50 and 250° C, wherein the catalytic system is formed from at least:

a) one nonhalogenated palladium source, and

b) at least one hydrogenophosphonium salt of formula:

$[HPR_1R_2R_3]^+Y^-$

and, where appropriate, a hydracid HY, $R_1$, $R_2$, $R_3$ and $Y^-$ having the meaning given in claim 1.

**3.** Process as claimed in claim 1 or 2, wherein the catalytic system is formed in a solvent medium.

**4.** Process as claimed in any one of the preceding claims, wherein $Y^-$, the anion associated with the hydracid is chosen from the group consisting of the anions:

$C_nF_{2n+1}CO_2^-$, $C_nF_{2n+1}SO_3^-$, 1/2 $SO_4^=$, $ClO_4^-$, $PF_6^-$, $SbF_6^-$, $BF_4^-$ and 1/2 $SiF_6^=$,

n being an integer of between 1 and 4 inclusive.

**5.** Process as claimed in any one of the preceding claims, wherein $Y^-$, the anion associated with the hydracid is a tetrafluoroborate anion.

**6.** Process as claimed in any one of the preceding claims, wherein the molar ratio of phosphorus to palladium is between 1 and 3.

**7.** Process according to any one of the preceding claims, wherein the molar ratio of $H^+$ ions to palladium is between 1 and 10.

**8.** Process as claimed in any one of claims 1 and 3 to 7 above, wherein the phosphine used is tributylphosphine.

**9.** Process as claimed in any one of claims 2 to 8 above, wherein the hydrogenophosphonium salt used is hydrogenotributylphosphonium tetrafluoroborate.

**10.** Process as claimed in any one of the preceding claims, wherein the palladium source is chosen from amongst compounds of palladium(O) of general formula (III):

$Pd_x(dba)_y$ (III)

in which:
x is equal to 1 or 2,
dba represents a dibenzylideneacetone coordinate,
y is equal to 2 or 3, y necessarily being equal to 3 when x equals 2.

**11.** Process as claimed in any one of claims 1 to 9 above, wherein the palladium source is palladium acetate.

**12.** Process as claimed in any one of claims 1 to 9 above, wherein the palladium source is palladium acetylacetonate.

**Patentansprüche**

**1.** Verfahren zum katalytischen Dimerisieren eines niederen Alkylacrylats durch in Berührungbringen eines niederen Alkylacrylats mit einem katalytischen System, das Palladium oder eine Palladiumverbindung enthält, bei einer Temperatur von 50 bis 250 °C, dadurch gekennzeichnet, daß das katalytische System gebildet wird, ausgehend von mindestestens

a) einer nicht halogenierten Quelle für Palladium

b) einer Verbindung von Phosphor (III) der allgemeinen Formel (I):

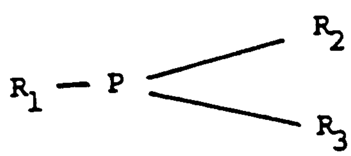

(I)

in der:
$R_1$, $R_2$ und $R_3$ jeweils eine Alkyl-, Cycloalkyl-, Aryl-, Alkoxy-, Cycloalkoxy- oder Aryloxygruppe bedeutet, eine der Gruppen $R_1$, $R_2$ oder $R_3$ außerdem eine einwertige Gruppe der allgemeinen Formel (II):

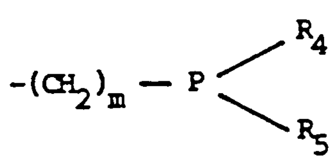

(II)

bedeuten kann, in der
- m eine ganze Zahl von 1 bis 4 ist
- $R_4$ und $R_5$ jeweils für eine Alkyl-, Cycoalkyl-, Aryl-, Alkoxy-, Cycloalkoxy- oder Aryloxygruppe stehen und

c) mindestens einer Wasserstoffsäure HY deren Anion $Y^-$ keinen Koordinationscharakter gegenüber den Palladiumionen besitzt.

**2.** Verfahren zum katalytischen Dimerisieren eines niederen Alkylacrylats durch Inberührungbringen eines niederen Alkylacrylats mit einem Katalysatorsystem, das Palladium oder eine Palladiumverbindung umfaßt, bei einer Temperatur von 50 bis 250 °C, dadurch gekennzeichnet, daß das Katalysatorsystem gebildet wird ausgehend von mindestens:

a) einer nicht halogenierten Quelle für Palladium
und
b) mindestens einem Hydrogenophosphoniumsalz der Formel:

$[HPR_1R_2R_3]^+Y^-$

und gegebenenfalls einer Wasserstoffsäure HY, wobei $R_1$, $R_2$, $R_3$ und $Y^-$ die in Anspruch 1 angegebene Bedeutung haben.

3. Verfahren nach Anspruch 1 oder 2, dadurch gekennzeichnet, daß das Katalysatorsystem in (einem) Lösungsmittelmedium gebildet wird.

4. Verfahren nach einem der vorangehenden Ansprüche, dadurch gekennzeichnet, daß $Y^-$, das Anion der Wasserstoffsäure, ausgewählt wird aus der Gruppe folgender Anionen:

$$C_nF_{2n+1}CO_2^-,\ C_nF_{2n+1}SO_3^-,\ 1/2\ SO_4^{=},\ ClO_4^-,\ PF_6^-,\ SbF_6^-,\ BF_4^-,\ 1/2\ SiF_6^{=},$$

wobei n eine ganze Zahl von 1 bis 4 ist.

5. Verfahren nach einem der vorangehenden Ansprüche, dadurch gekennzeichnet, daß $Y^-$, das Anion der Wasserstoffsäure, ein Tetrafluoroborat-Anion ist.

6. Verfahren nach einem der vorangehenden Ansprüche, dadurch gekennzeichnet, daß das Molverhältnis von Phosphor zu Palladium 1 bis 3 beträgt.

7. Verfahren nach einem der vorangehenden Ansprüche, dadurch gekennzeichnet, daß das Molverhältnis der Wasserstoffionen zu Palladium 1 bis 10 beträgt.

8. Verfahren nach einem der vorhergehenden Ansprüche 1 und 3 bis 7, dadurch gekennzeichnet, das das verwendete Phosphin Tributylphosphin ist.

9. Verfahren nach einem der vorangehenden Ansprüche 2 bis 8, dadurch gekennzeichnet, daß das eingesetzte Hydrogenophosphoniumsalz das Hydrogenotributylphosphonium-tetrafluoroborat ist.

10. Verfahren nach einem der vorangehenden Ansprüche, dadurch **gekennzeichnet,** daß die Palladiumquelle ausgewählt wird aus den Verbindungen von Palladium (0) der allgemeinen Formel (III):

$Pd_x(dba)_y$ (III)

in der x = 1 oder 2
dba ein Dibenzylidenaceton-Koordinat bedeutet,
Y = 2 oder 3, wobei y notwendigerweise gleich 3 ist, wenn x den Wert 2 hat.

11. Verfahren nach einem der vorangehenden Ansprüche 1 bis 9, dadurch gekennzeichnet, daß die Palladiumquelle Palladiumacetat ist.

12. Verfahren nach einem der vorangehenden Ansprüche 1 bis 9, dadurch gekennzeichnet, daß die Palladiumquelle Palladiumacetylacetonat ist.